# EUROPEAN PATENT APPLICATION

(11) **EP 3 878 976 A1**
(43) Date of publication of application: **15.09.2021**
(21) Application number: 19882355.1
(22) Date of filing: 06.11.2019
(51) Int. Cl.: C12Q 1/6881, C12N 5/0775

(54) **BIOMARKER FOR PREDICTING ABILITY OF MESENCHYMAL STEM CELLS TO PROLIFERATE AND MIGRATE**

(30) Priority: 09.11.2018 KR 20180137579
(71) Applicant: Corestem Co., Ltd., Cheongju-si, Chungcheongbuk-do 28420 (KR)
(72) Inventor: KIM, Kyung Suk, Seoul 02838 (KR); LEE, Tae Yong, Gyeonggi-do 17128 (KR)
(74) Representative: J A Kemp LLP
(86) International application number: PCT/KR2019/014959
(87) International publication number: WO 2020/096339

(57) **Abstract**

The present invention relates to a biomarker for prediction of proliferation and migration capacity of mesenchymal stem cells and a use thereof, and more specifically, the present invention relates to a biomarker for prediction of proliferation or migration capacity of mesenchymal stem cells including peroxiredoxin 6 and a use thereof.

According to the present invention, by measuring the expression level of the biomarker, the proliferation and migration capacity of mesenchymal stem cells can be predicted.

<110>	CoreStem CO.,LTD.

<120>	BIOMARKER FOR PREDICTION OF PROLIFERATION AND MIGRATION CAPACITY
	OF MESENCHYMAL STEM CELL AND USE THEREOF

<130>	PCT5040318

<150>	KR 10-2018-0137579
<151>	2018-11-09

<160>	24

<170>	KoPatentIn 3.0

<210>	1
<211>	25
<212>	DNA
<213>	Artificial Sequence

<220>
<223>	CCL2_F primer


<400>	1
atgaaagtct ctgccgccct tctgt	25


<210>	2
<211>	25
<212>	DNA
<213>	Artificial Sequence

<220>
<223>	CCL2_R primer


<400>	2
agtcttcgga gtttgggttt gcttg	25


<210>	3
<211>	24
<212>	DNA
<213>	Artificial Sequence

<220>
<223>	CCL3_F primer


<400>	3
atgcaggtct ccactgctgc cctt	24


<210>	4
<211>	25
<212>	DNA
<213>	Artificial Sequence

<220>
<223>	CCL3_R primer


<400>	4

gcactcagct ccaggtcgct gacat                                                25

<210>    5
<211>    20
<212>    DNA
<213>    Artificial Sequence

<220>
<223>    CCL4_F primer

<400>    5
ccaaaccaaa agaagcaagc                                                      20

<210>    6
<211>    20
<212>    DNA
<213>    Artificial Sequence

<220>
<223>    CCL4_R primer

<400>    6
agaaacagtg acagtggacc                                                      20

<210>    7
<211>    24
<212>    DNA
<213>    Artificial Sequence

<220>
<223>    CCL5_F primer

<400>    7
gagtatttct acaccagtgg caag                                                 24

<210>    8
<211>    21
<212>    DNA
<213>    Artificial Sequence

<220>
<223>    CCL5_R primer

<400>    8
tcccgaaccc atttcttctc t                                                    21

<210>    9
<211>    20
<212>    DNA
<213>    Artificial Sequence

<220>
<223>    CXCL10_F primer

<400>    9
cctgcttcaa atatttccct                                                    20


<210>    10
<211>    20
<212>    DNA
<213>    Artificial Sequence

<220>
<223>    CXCL10_R primer


<400>    10
ccttcctgta tgtgtttgga                                                    20


<210>    11
<211>    22
<212>    DNA
<213>    Artificial Sequence

<220>
<223>    CXCL12_F primer


<400>    11
atgaacgcca aggtcgtggt cg                                                 22


<210>    12
<211>    19
<212>    DNA
<213>    Artificial Sequence

<220>
<223>    CXCL12_R primer


<400>    12
tgttgttgtt cttcagccg                                                     19


<210>    13
<211>    25
<212>    DNA
<213>    Artificial Sequence

<220>
<223>    PRDX6 _F primer


<400>    13
gtcgccatgc ccggaggtct gcttc                                              25


<210>    14
<211>    26
<212>    DNA
<213>    Artificial Sequence

<220>

<223>    PRDX6 _R primer

<400>    14
aattggcagc tgacatcctc tggctc                                          26

<210>    15
<211>    20
<212>    DNA
<213>    Artificial Sequence

<220>
<223>    COX-2 _F primer

<400>    15
tccttgctgt tcccacccat                                                 20

<210>    16
<211>    20
<212>    DNA
<213>    Artificial Sequence

<220>
<223>    COX-2 _R primer

<400>    16
catcatcaga ccaggcacca                                                 20

<210>    17
<211>    20
<212>    DNA
<213>    Artificial Sequence

<220>
<223>    iNOS_F primer

<400>    17
acgtgcgtta ctccaccaac                                                 20

<210>    18
<211>    20
<212>    DNA
<213>    Artificial Sequence

<220>
<223>    iNOS_R primer

<400>    18
catagcggat gagctgagca                                                 20

<210>    19
<211>    21
<212>    DNA
<213>    Artificial Sequence

<220>
<223>    IDO_F primer


<400>    19
agcctgatct catagagtct g                                                21


<210>    20
<211>    20
<212>    DNA
<213>    Artificial Sequence

<220>
<223>    IDO_R primer


<400>    20
ttactgcagt ctccatcacg                                                  20


<210>    21
<211>    18
<212>    DNA
<213>    Artificial Sequence

<220>
<223>    TGF-beta_F primer


<400>    21
cagatcctgt ccaagctg                                                    18


<210>    22
<211>    18
<212>    DNA
<213>    Artificial Sequence

<220>
<223>    TGF-beta_R primer


<400>    22
tcggagctct gatgtgtt                                                    18


<210>    23
<211>    20
<212>    DNA
<213>    Artificial Sequence

<220>
<223>    beta-actin_F primer


<400>    23
gtggggcgcc ccaggcacca                                                  20


<210>    24
<211>    20

<212>    DNA
<213>    Artificial Sequence

<220>
<223>    beta-actin_R primer


<400>    24
ctccttaatg tcacgcacga                                                    20

## Description

### CROSS-REFERENCE TO RELATED APPLICATION

This application claims priority to and the benefit of Korean Patent Application No 10-2018-0137579, filed on November 9, 2018, the disclosure of which is incorporated herein by reference in its entirety.

### [Technical Field]

The present invention relates to a biomarker for prediction of proliferation and migration capacity of mesenchymal stem cells and a use thereof, and more specifically, the present invention relates to a biomarker for prediction of proliferation or migration capacity of mesenchymal stem cells including peroxiredoxin 6 and a use thereof.

### [Background Art]

Stem cells are collectively referred to as undifferentiated cells at a stage before being differentiated into each cell constituting tissues, and differentiation into specific cells proceeds by specific differentiation stimuli (environment). Unlike differentiated cells in which cell division is stopped, stem cells can produce cells which are identical to themselves (self-renewal) by cell division, and thus, they have the property of proliferation (expansion). In addition, when a differentiation stimulus is applied, they differentiate into specific cells, and since they can be differentiated into other cells by different environments or different differentiation stimuli, they are characterized in having plasticity of differentiation.

The pluripotency of these stem cells provides a good *in vitro* experimental model for the study of human developmental processes. New drug development can be facilitated by performing drug tests and toxicity tests on homogeneous human tissues or cells obtained from stem cells. Furthermore, it is possible to obtain a large amount of cells or tissues that can replace damaged tissues, which can be used to treat incurable diseases.

Mesenchymal stem cells are cells that maintain sternness and self-renewal and have the capacity to differentiate into various mesenchymal tissues (plasticity). These cells can be extracted from bone marrow, adipose tissue, umbilical cord blood, synovial membrane, trabecular bone, infrapatellar fat pad, and the like. Mesenchymal stem cells inhibit the activity and proliferation of T lymphocytes and B lymphocytes, suppress the activity of natural killer cells (NK cells), and have immunomodulatory capacity to regulate the functions of dendritic cells and macrophages, and thus, these are cells capable of allotransplantation and xenotransplantation. In addition, mesenchymal stem cells have the ability to differentiate into various connective tissues such as cartilage, bone tissue, ligaments, bone marrow matrix, and the like. In particular, adipose-derived mesenchymal stem cells derived from adipose tissue are subdivided into names such as adipose-derived stem/stromal cells (ASC) or adipose-derived adult stem cells (ADAS), and these can be applied to the production of biomaterials to treat soft tissue defects caused by trauma, tumor removal surgery, burns, and the like.

Meanwhile, peroxiredoxin (PRDX) is a peroxidase that reduces peroxide, and PRDX is expressed in many tissues and exists in large quantities, and thus, it is expected to play an important role *in vivo.* In mammals, PRDX is present in a large amount of 0.2% to 0.4% of the total protein in the cytosol. In mammals, 12 isoforms have been reported, and these are representatively divided into 6 types (PRDX1 to 6). PRDX1 to 5 are structures with 2-cysteine, and thioredoxin is used as a reducing agent. In addition, PRDX6 is a structure with 1-cysteine, and glutathione is used as a reducing agent.

Until now, it has been known that peroxiredoxin 1 is used as a lung cancer diagnostic protein marker or breast cancer diagnostic marker, and a technique of using peroxiredoxin 2 for cancer diagnosis and therapy and a use of peroxiredoxin 6 as a target for cancer invasion or metastasis (Korean Patent Laid-Open No. 10-2009-0095304) and the like have been disclosed. However, there has been no report on the use of peroxiredoxin 6 as a biomarker for prediction of the proliferation and migration capacity of mesenchymal stem cells.

### Related Art Documents

Korean Patent Laid-Open No. 10-2009-0095304

### [Disclosure]

### [Technical Problem]

The present invention provides a biomarker for prediction of proliferation and migration capacity of mesenchymal stem cells, including peroxiredoxin 6.

In addition, the present invention provides a composition for prediction of proliferation and migration capacity of mesenchymal stem cells, including a agent for measuring the expression level of the biomarker.

In addition, the present invention provides a kit for prediction of proliferation and migration capacity of mesenchymal stem cells, including the composition.

In addition, the present invention provides a method for providing information for prediction of proliferation and migration capacity of mesenchymal stem cells.

However, the technical problems to be achieved by the present invention are not limited to the problems mentioned above, and other problems not mentioned will be clearly understood by those skilled in the art from the following description.

### [Technical Solution]

The present invention provides a biomarker for prediction of proliferation and migration capacity of mesenchymal stem cells, including peroxiredoxin 6.

The peroxiredoxin 6 may regulate integrin β3 of mesenchymal stem cells.

Cell proliferation and migration capacity may be increased when the peroxiredoxin 6 is overexpressed.

In addition, the present invention provides a composition for prediction of proliferation and migration capacity of mesenchymal stem cells, including a agent for measuring the expression level of the biomarker.

Provided is a composition for prediction of proliferation and migration capacity of mesenchymal stem cells, in which the agent for measuring the expression level of the biomarker includes a primer pair, a probe or an antisense nucleotide that specifically binds to the biomarker.

In addition, the present invention provides a kit for prediction of proliferation and migration capacity of mesenchymal stem cells, including the composition.

The kit may be an RT-PCR kit, a competitive RT-PCR kit, a real-time RT-PCR kit, a quantitative RT-PCR kit, or a DNA chip kit.

In addition, the present invention provides a method for providing information for prediction of proliferation and migration capacity of mesenchymal stem cells, including measuring the expression level of the biomarker from a biological sample of a subject to compare with the expression level of the corresponding biomarker of a comparative control group sample.

The method for measuring the expression level of the biomarker may be reverse transcriptase polymerase chain reaction (RT-PCR), competitive reverse transcriptase polymerase chain reaction (competitive RT-PCR), real-time quantitative reverse transcriptase polymerase chain reaction (real-time quantitative RT-PCR), quantitative RT-PCR, an RNase protection method, Northern blotting or DNA chip technology, Northern blotting, immunohistochemical staining, immunoprecipitation assay, complement fixation assay, or immunofluorescence.

In addition, the present invention provides a method for screening mesenchymal stem cells with excellent cell proliferation and migration capacity, by measuring the expression level of peroxiredoxin 6.

### [Advantageous Effects]

The present invention relates to a biomarker for prediction of proliferation and migration capacity of mesenchymal stem cells including peroxiredoxin 6, and by measuring the expression level of the biomarker, the proliferation and migration capacity of mesenchymal stem cells can be predicted. In addition, by predicting the proliferation and migration capacity of mesenchymal stem cells, stem cells that are administered for therapy can reach the target tissue or site and increase the efficacy of therapy more than before, and thus, the efficacy of cell therapy can be improved in this aspect.

### [Description of Drawings]

FIG. 1 is an analysis of the expression of PRDX6 in the spleen and kidney of MRL/lpr mice (* p < 0.01).
FIG. 2 is an analysis of the expression level of PRDX6 in MSC.
FIG. 3 is an analysis of the effect of PRDX6-KD (knockdown) hMSC on cell proliferation and apoptosis (* p < 0.01).
FIG. 4 is an analysis of the effect of PRDX6-KD hMSC on hydrogen peroxide (* p < 0.01).
FIG. 5 is an analysis of the effect of PRDX6-KD hMSC on cell adhesion and spreading (* p < 0.01).
FIG. 6 is an analysis of the effect of PRDX6-KD hMSC on motility and invasion (* p < 0.01).
FIG. 7 is an analysis of the effect of PRDX6-KD hMSC on migration (* p < 0.01).
FIG. 8 is an analysis of the association between PRDX6-KD hMSC, integrin, and MMP
FIG. 9 is an analysis of the effect of PRDX6-KD hMSC on immunosuppressive capacity.
FIG. 10 is an analysis of the characteristics of PRDX6-transgenic mMSC.
FIG. 11 is an analysis of the effect of PRDX6-transgenic mMSC on cell proliferation.
FIG. 12 is an analysis of the effect of PRDX6-transgenic mMSC on motility and invasion (* p < 0.01).
FIG. 13 is an analysis of the effect of PRDX6-transgenic mMSC on immunosuppressive capacity (* p < 0.01).
FIG. 14 is an analysis of the association between PRDX6-KD hMSC and PGE2 (* p <0.01).

### [Modes of the Invention]

Hereinafter, the present invention will be described in more detail.

### Biomarker for prediction of proliferation and migration capacity of mesenchymal stem cells

The present invention provides a biomarker for prediction of proliferation and migration capacity of mesenchymal stem cells, including peroxiredoxin 6. The peroxiredoxin 6 may regulate integrin β3 of mesenchymal stem cells. When peroxiredoxin 6 is overexpressed, cell proliferation and migration capacity may be increased, and when peroxiredoxin 6 is underexpressed, cell proliferation and migration capacity may be reduced.

As used herein, the term "overexpression" refers to a case where the expression level of a target nucleotide sequence in the sample to be investigated (e.g., human bone marrow-derived mesenchymal stem cells) is higher than the average expression value of wild-type mesenchymal stem cells (preferably, 1.5 times or more).

The mesenchymal stem cells may be stem cells derived from bone marrow, fat, umbilical cord blood, placenta, umbilical cord blood, or blood.

### Composition for prediction of proliferation and migration capacity of mesenchymal stem cells

The present invention provides a composition for prediction of proliferation and migration capacity of mesenchymal stem cells, including a agent for measuring the expression level of the biomarker.

Specifically, a agent that measures the expression level of the biomarker may include a primer pair, a probe, or an antisense nucleotide that specifically binds to the biomarker. The composition according to the present invention may predict the proliferation and migration capacity of mesenchymal stem cells through PCR amplification using the sense and antisense primers of the polynucleotide of peroxiredoxin 6 to produce the desired product, and PCR conditions, sense and antisense primer lengths may be modified based on those known in the art. In addition, the primers of the present invention may be chemically synthesized by using a phosphoramidite solid support method or other well-known methods, and such nucleic acid sequences may also be modified by using many means known in the art. Non-limiting examples of such modifications include methylation, encapsulation, substitution with one or more homologs of natural nucleotides, and modification between nucleotides, such as uncharged linkages (*e.g.,* methyl phosphonate, phosphotriester, phosphor-amidates, carbamates, *etc.*) or modification to charged linkages (*e.g.,* phosphorothioates, phosphorodithioates, *etc.*)*.*

### Kit for prediction of proliferation and migration capacity of mesenchymal stem cells

The present invention provides a kit for prediction of proliferation and migration capacity of mesenchymal stem cells including the composition. The kit may be an RT-PCR kit, a competitive RT-PCR kit, a real-time RT-PCR kit, a quantitative RT-PCR kit, or a DNA chip kit.

Specifically, the kit for measuring the expression level of the biomarker may be a kit including essential elements necessary for performing RT-PCR. The RT-PCR kit may include test tubes or other suitable containers, reaction buffers, deoxynucleotides (dNTPs), Taq-polymerase and reverse transcriptase, DNase, RNase inhibitors, DEPC-water, sterilized water, and the like, in addition to each primer pair specific for a marker gene.

In addition, the kit of the present invention may be a kit for detecting a gene for prediction of the proliferation and migration capacity of mesenchymal stem cells including essential elements necessary for performing a DNA chip. The DNA chip kit may include a substrate to which cDNA corresponding to a gene or a fragment thereof is attached as a probe, and the substrate may include cDNA corresponding to a quantitative control gene or a fragment thereof.

### Method for providing information for prediction of the proliferation and migration capacity of mesenchymal stem cells

The present invention provides a method for providing information for prediction of the proliferation and migration capacity of mesenchymal stem cells, including measuring the expression level of the biomarker from a biological sample of a subject to compare with the expression level of the corresponding biomarker of a comparative control group sample.

The method for measuring the expression level of the biomarker may be reverse transcriptase polymerase chain reaction (RT-PCR), competitive reverse transcriptase polymerase chain reaction (competitive RT-PCR), real-time quantitative reverse transcriptase polymerase chain reaction (real time quantitative RT-PCR), quantitative RT-PCR, RNase protection method, Northern blotting or DNA chip technology, Northern blotting, immunohistochemical staining, immunoprecipitation assay, complement fixation assay, or immunofluorescence.

As used herein, the term "biological sample" refers to any sample obtained from a subject in which the expression of the biomarker of the present invention may be detected. For example, it may be obtained from bone marrow, adipose tissue, peripheral blood, or the like, but is not limited thereto, and it may be prepared by treatment using a method conventionally used in the technical field of the present invention.

### Method for screening mesenchymal stem cells with excellent cell proliferation and migration capacity

The present invention provides a method for screening mesenchymal stem cells with excellent cell proliferation and migration capacity by measuring the expression level of peroxiredoxin 6. When peroxiredoxin 6 is overexpressed, the proliferation, migration, and adhesion capacities of mesenchymal stem cell are increased, and when peroxiredoxin 6 is underexpressed, the proliferation, migration, and adhesion capacities of mesenchymal stem cells are reduced.

Hereinafter, the present invention will be described in more detail through exemplary embodiments. The objects, features, and advantages of the present invention will be readily understood through the following exemplary embodiments. The present invention is not limited to the exemplary embodiments described herein, and may be embodied in other forms. The exemplary embodiments introduced herein are provided to sufficiently convey the spirit of the present invention to those skilled in the art to which the present invention pertains. Therefore, the present invention should not be limited by the following exemplary embodiments.

### <Example>

### Materials and experimental methods

### Materials

Female MRL/MpJ-Faslpr/J (hereinafter, referred to as MRL/lpr) mice were purchased from Jackson Laboratory, USA. The breeding environment of the mice was maintained at a specific pathogen-free (SPF) state at a temperature of 21°C to 24°C, a humidity of 40% to 60%, and a contrast cycle of 12 hours (light on: 08 o'clock, light off: 20 o'clock). The solid feed and drinking water were sterilized and freely fed, and all experimental animals were used in the experiment after being acclimated in the animal room for one week.

Human mesenchymal stem cells (MSC, hereinafter, referred to as hMSC) and mouse MSC (hereinafter, referred to as mMSC) were obtained from bone marrow (BM) cells from human or mouse tibiae and femurs. The hMSC and BM cells were cultured under a condition of 37°C and 7% CO₂ in the CSBM-A06 medium (CoreStem, Co. Ltd., Korea) including 10% fetal bovine serum (FBS) and 1% penicillin streptomycin in the medium.

PRDX6-knockdown hMSC was cultured for 48 hours by using a phenomenon of sequence-specific inhibition of gene expression by a 12 mer to 21 mer dsRNA called small interfering RNA (siRNA) (made by Bioneer, Korea) to induce the PRDX6-knockdown reaction.

### IFN-γ ELISA

The IFN-γ measurement was performed according to the test method provided by R&D (#DY285-05). The capture antibody and coating buffer were diluted at a ratio of 1: 250 in 96 wells for ELISA and coated at 4°C for 18 hours. After washing twice using a 200 µL of a wash solution, 250 µL of a blocking buffer was placed in a well, and then, it was reacted at room temperature for 2 hours. Afterwards, washing was performed twice using 200 µL of a wash solution, and the sample was prepared by diluting a specimen in an assay buffer (1X). In each well, 90 µL of the assay buffer (1X), 50 µL of a detection antibody, and 10 µL of a standard or the sample were added and reacted at room temperature for 3 hours. After the reaction was completed, washing was performed using a wash solution. 100 µL of a substrate solution was added to each well, followed by reacting for 5 minutes, and 100 µL of a stop solution was added to stop the reaction. Finally, the value was measured at 450 nm to 570 nm.

### RNA isolation using the Trizol method

Cells were treated with Trypsin-EDTA (Gibco) at 0.125% at a time point when the confluency of 70% to 80% was reached, followed by culturing under a condition of 5% CO₂ and 37 °C. After 24 hours of material treatment, the cells were recovered, and the total RNA was isolated from the cells using the TRIZOL reagent (Invitrogen, MD, USA), and the total RNA was quantified by absorbance measurement at 260 nm.

### Western blot

Cells were lysed in ice using a cell lysis buffer (Cell Signaling Technology, Danvers, MA, USA). Proteins were obtained by centrifugation at 12,000 rpm for 15 minutes at 4°C. The proteins were quantified using the Bradford reagent, and electrophoresis was performed at 75V with 20 µg of the protein by using an SDS-polyacrylamide gel. After the SDS-polyacrylamide gel was transferred to a PVDF membrane at 95V for 90 minutes, 5% nonfat dry milk was added to TBS (TTBS) including 0.5% Tween 20 and blocked for 1 hour. After blocking, the membrane was placed in 5% BSA/TTBS including a primary antibody and shaken at 4°C for one day. The next day, after washing the membrane with TTBS, the membrane was placed in 5% BSA/TTBS including a secondary antibody, and the secondary antibody was attached at room temperature for 90 minutes. After washing the membrane with TTBS and reacting the membrane with enhanced chemiluminescence (ECL, Amersham Pharmacia Biotech, Piscataway, NJ, USA), the degree of protein expression was measured using a ChemiDoc™ XRS+ machine (Bio-Rad, CA, USA).

### Reverse transcriptase-PCR

A total RNA concentration of 0.3 µg was used, and it was synthesized at 42°C for 1 hour and at 95°C for 5 minutes. Polymerase chain reaction was performed using 3 µL of synthesized cDNA and 10 pM of primer. For the basic process of this reaction, the pre-denaturing phase was performed at 94°C for 5 minutes, the denaturing phase was performed at 94°C for 30 seconds, the annealing phase was performed at 56°C for 30 seconds, the elongation phase was performed at 72°C for 1 minute, and the post-elongation phase was performed at 72°C for 5 minutes. Electrophoresis was performed by making a 1% agarose gel.

### RT-PCR

The expression levels of mRNA for PRDX6, COX-2, iNOS, IDO, TGF-β, CCL2, CCL4, CCL5, CXCL10, and CXCL12 were analyzed by quantitative real-time PCR (qPCR). The relative mRNA amount of each sample was calculated based on a threshold cycle (Ct) compared to a threshold cycle (Ct) of β-actin, which is a housekeeping gene.

### Primer sequence information

The information of the used primers is as follows. CCL2 forward primer 5-'ATG AAA GTC TCT GCC GCC CTT CTG T-3', CCL2 reverse primer 5-'AGT CTT CGG AGT TTG GGT TTG CTT G-3', CCL3 forward primer 5'-ATG CAG GTC TCC ACT GCT GCC CTT-3', CCL3 reverse primer 5'-GCA CTC AGC TCC AGG TCG CTG ACA T-3', CCL4 forward primer 5'-CCA AAC CAA AAG AAG CAA GC-3', CCL4 reverse primer 5'-AGA AAC AGT GAC AGT GGA CC-3', CCL5 forward primer 5'-GAG TAT TTC TAC ACC AGT GGC AAG-3', CCL5 reverse primer 5'-TCC CGA ACC CAT TTC TTC TCT-3', CXCL10 forward primer 5'- CCT GCT TCA AAT ATT TCC CT-3', CXCL10 reverse primer 5'-CCT TCC TGT ATG TGT TTG GA-3', CXCL12 forward primer 5'-ATG AAC GCC AAG GTC GTG GTC G-3', CXCL12 reverse primer 5 '-TGT TGT TGT TCT TCA GCC G-3', PRDX6 forward primer 5'-GTC GCC ATG CCC GGA GGT CTG CTT C-3', PRDX6 reverse primer 5'-AAT TGG CAG CTG ACA TCC TCT GGC TC-3' , COX-2 forward primer 5'-TCC TTG CTG TTC CCA CCC AT-3 ', COX-2 reverse primer 5'-CAT CAT CAG ACC AGG CAC CA-3', iNOS forward primer 5'-ACG TGC GTT ACT CCA CCA AC-3 ', iNOS reverse primer 5 '-CAT AGC GGA TGA GCT GAG CA-3', IDO forward primer 5'-AGCC TGA TCT CAT AGA GTC TG-3', IDO reverse primer 5'-TTA CTG CAG TCT CCA TCA CG-3', TGF-β forward primer 5'-CAG ATC CTG TCC AAG CTG-3 ', TGF-β reverse primer 5'-TCG GAG CTC TGA TGT GTT-3', β-actin forward primer 5'-GTG GGG CGC CCC AGG CAC CA-3', β-actin reverse primer 5'-CTC CTT AAT GTC ACG CAC GA-3'.

### Annexin V staining

After washing the cells twice with cold PBS, the cells were released in a binding buffer at a concentration of 1 × 10⁶ cells/mL. Then, after transferring an amount of 100 µL to a 5 mL culture tube, the cells were stained by passing through an incubation process for 15 minutes at 25°C room temperature, and under a dark condition using 5 µL of the Annexin V Apoptosis Detection Kit (BD Pharmingen, USA). Then, after adding 400 µL of a binding buffer solution, it was measured using flow cytometry (Canto II, BD Bioscience).

### [³H]-Thymidine uptake analysis

After culturing for 54 hours in a 37°C incubator supplied with 5% CO₂, [³H]-thymidine (Perkin Elmer, MA, USA) was treated to 1 µCi/well. After culturing for 18 hours, the amount of [³H]-thymidine in the cells was measured with a Wallac Microbeta scintillation counter (Wallac, Turky, Finland) using a cell harvester (Inotech, Dottikonm, Switzerland).

### Time-lapse imaging

MSC at 3 × 10⁵ cells/well was dispensed for 24 hours using µ-dish^{35mm} culture dishes (ibidi GmbH, Martinsried, Germany) for imaging photograph to confirm cell migration. After the cells were stabilized, inserts were removed and photographed using Biostation IM-Q (Nikon, Tokyo, Japan). In this experiment, photographing was performed for 12 hours at 2-minute intervals, and data analysis was performed by using the Imaris 7.2 software (Bitplane Inc., South Windsor, CT, USA).

### Chemotaxis assay

For the migration of MSC, 24-transwell plates with a 5 µm insert upper well were used (Costar, Corning, NY, USA). A medium containing a specific chemokine was dispensed into the lower chamber, and MSC at 1 × 10⁵ cells/well was dispensed into the upper chamber. After culturing for 1.5 hours in a 37°C incubator supplied with 5% CO₂, the number of cells of MSC that moved to the lower chamber was counted using flow cytometry to express the value.

### Measurements of MSC motility and invasion

For the measurement of MSC motility, after treating siRNA in hMSC (1 × 10⁴ cells/well), the cells were cultured by dispensing for 24 hours in a transwell plate. Afterwards, crystal violet (Sigma, USA) staining was performed for analysis. For the measurement of MSC invasion, 15% Matrigel was coated on a transwell plate for 24 hours, and then, hMSC (1 × 10⁴ cells/well) was dispensed and cultured for 24 hours. Afterwards, crystal violet staining was performed for analysis.

### MSC adhesion and spreading measurement

For the measurement of MSC spreading, a control group and hMSC with suppressed PRDX6 expression were stained with CMFDA (Invitrogen, green) and CMTMR (Invitrogen, red), respectively. Afterwards, each cell was cultured by dispensing hMSC (0.2 × 10⁵ cells/well) in culture-Dish35^{mm}, and time-lapse imaging was filmed for 4 hours at 2-minute intervals. Snapshots of movies were taken for each time period to observe the adhesion and spreading of hMSC.

### Statistical analysis

Statistical analysis of experimental data was performed using the GraphPad Prism 5.0 (GraphPad, San Diego, CA, USA) software.

### Example 1. PRDX6 analysis in spleens and kidneys of MRL/lpr mice

The spleens and kidneys of 8-week-old and 22-week-old MRL/lpr mice were separated, and the total RNA was isolated using Trizol, followed by performing qPCR. The spleens and kidneys of 8-week-old and 22-week-old MRL/lpr mice were separated to obtain proteins, and then, a western blot was performed.

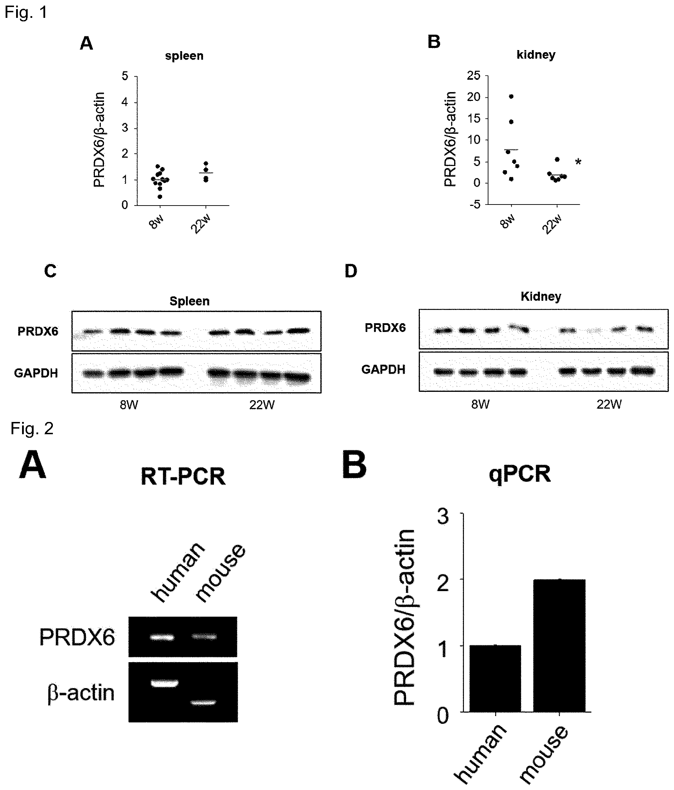

Since MRL/lpr mice begin to develop symptoms from 10 to 12 weeks of age and die within 20 weeks of age, the 8-week-old mice were judged to be in the early stage of the disease, and the 22-week-old mice were judged to be in the final stage of the disease. Then, the kidneys and spleens of 8-week-old and 22-week-old mice were separated, and the expression level of PRDX6 was analyzed, respectively. In the spleens of MRL/lpr mice, there was no difference between the expression level of the 8-week-old and the expression level of the 22-week-old (A and C in FIG. 1), and in the kidneys, the expression of PRDX6 was reduced when the condition was rather severe (B and D in FIG. 1). Through this, in conclusion, it was confirmed that PRDX6 was not suitable as a diagnostic and predictive biomarker for lupus disease.

### Example 2. Analysis of PRDX6 expressed in MSC

In human MSC and mouse MSC, after the total RNA was isolated using TRIzol, gene expression was confirmed through RT-PCR (A in FIG. 2) and qPCR (B in FIG. 2). It was confirmed that PRDX6 was present in a large amount in the tissues, and was also expressed in human MSC and mouse MSC through RT-PCR (A in FIG. 2) and qPCR (B in FIG. 2). Subsequently, additional experiments were conducted to confirm the functional aspects of PRDX6 of MSC.

### Example 3. Effect of PRDX6-KD (knockdown) hMSC on cell proliferation and apoptosis

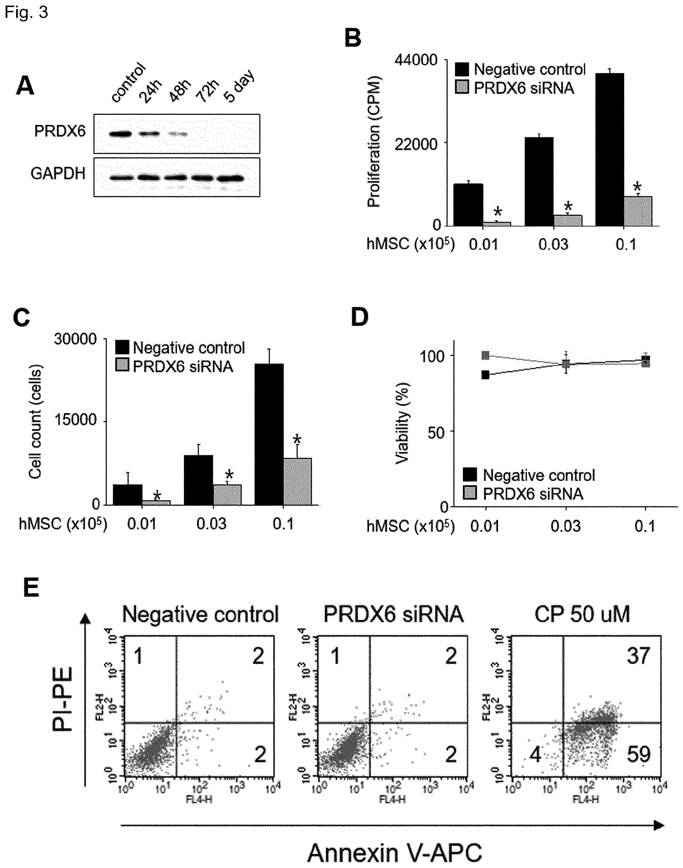

After siRNA was treated to hMSC in a time course, a western blot was performed (A in FIG. 3). The hMSC was added to a 96-well plate, and then cultured for 72 hours. Cell proliferation was measured through a thymidine uptake (B in FIG. 3) and cell count (C in FIG. 3), and cell viability was measured through trypan blue staining (D in FIG. 3). The hMSC was added, and after 72 hours, the cells were stained with annexin V and analyzed through the fluorescence activated cell sorter (FACS) (E in FIG. 3).

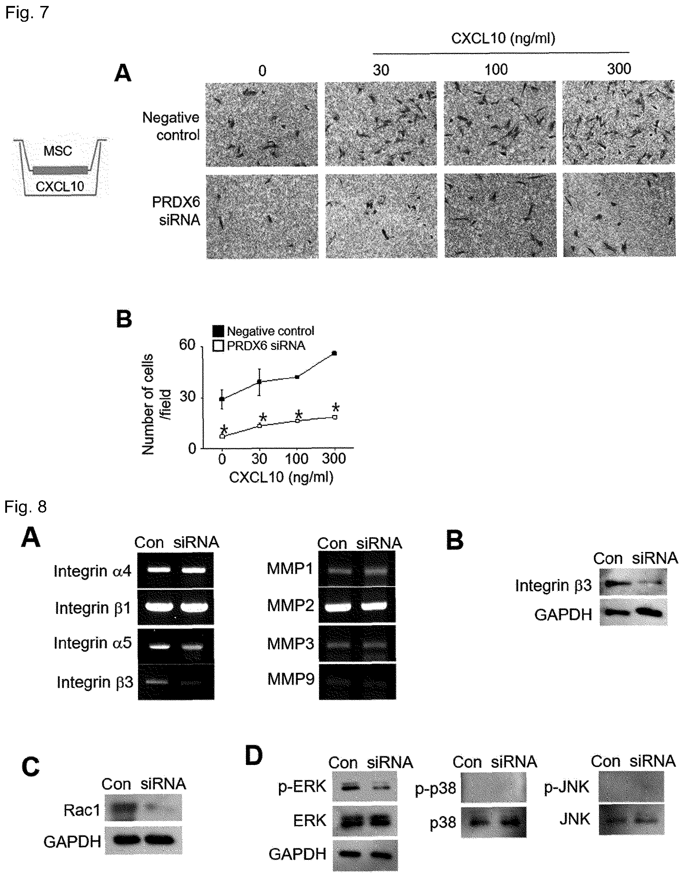

In order to determine the function of PRDX6 of hMSC, as a result of conducting an experiment after siRNA was treated to knock down (KD), it was confirmed that the expression of PRDX6 was reduced after 24 hours of siRNA treatment, and subsequent experiments were performed with siRNA treatment for 48 hours (A in FIG. 3). After knocking down PRDX6 of hMSC with siRNA, proliferation was measured through a thymidine uptake and cell count, and as a result, it was confirmed that the proliferation of hMSC in which PRDX6 was knocked down (KD) was suppressed compared to the control group (B and C in FIG. 8). In addition, as a result of measuring cell viability through trypan blue staining, it was confirmed that PRDX6 did not affect the viability of hMSC (D in FIG. 3). It is known that reactive oxygen species induces apoptosis of cells, and PRDX6 neutralizes hydrogen peroxide among reactive oxygen species. Accordingly, after knocking down (KD) PRDX6 of hMSC, an experiment was performed to determine whether apoptosis was observed. As a result of analysis through annexin V staining, it was confirmed that apoptosis was not induced in hMSC in which PRDX6 was knocked down (KD), and it was confirmed that cyclophosphamide (CP) which was used as a positive control group induced apoptosis in hMSC (E in FIG. 3). In conclusion, it was confirmed through this experiment that PRDX6 of hMSC has an effect on cell proliferation.

### Example 4. Analysis of the effect of PRDX6-KD (knockdown) hMSC on hydrogen peroxide

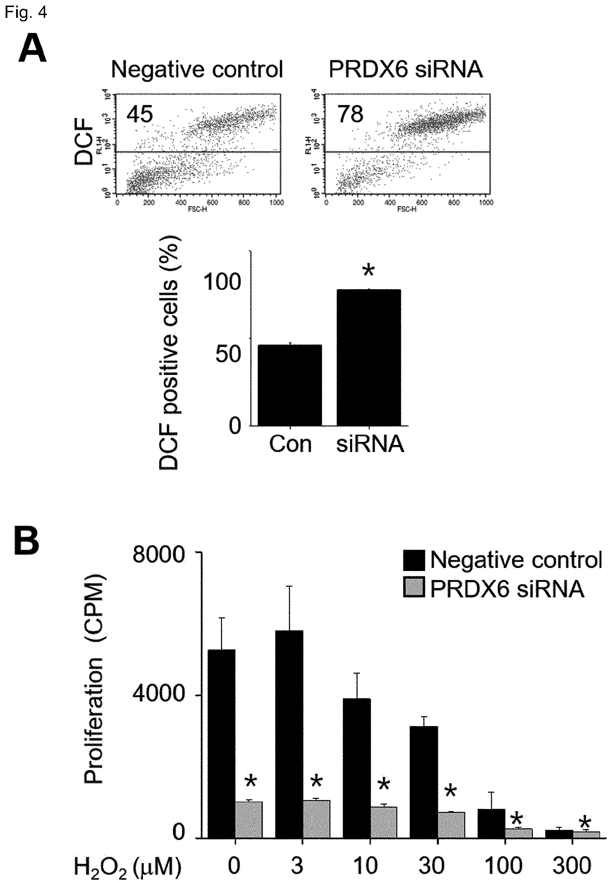

After siRNA was treated to hMSC, followed by culturing for 72 hours, dichlorodihydrofluorescein-diacetate (DCF-DA) was treated for 30 minutes, followed by FACS analysis (A in FIG. 4). In addition, after hMSC was added, hydrogen peroxide was treated, and cell proliferation was analyzed through a thymidine uptake after 72 hours (B in FIG. 4).

In order to determine the mechanism by which PRDX6 regulates hMSC proliferation, the amount of intracellular hydrogen peroxide was measured. DCF-DA is converted into DCF in the cell and is transformed into a substance that is fluorescent by intracellular hydrogen peroxide, which can be measured using FACS. After DCF-DA was treated to hMSC, the intracellular fluorescence value was confirmed by using FACS, and as a result, hMSC in which PRDX6 was knocked down (KD) had an increase in the fluorescence value by about 2 times compared to the control group (A in FIG. 4). In addition, after hydrogen peroxide was directly treated to hMSC, the degree of cell proliferation was measured, and as a result, it was confirmed that hydrogen peroxide reduced the proliferation of hMSC in which PRDX6 was knocked down and the control group of hMSC (B in FIG. 4). In conclusion, through this experiment, it can be confirmed that PRDX6 increases the proliferation of hMSC by removing hydrogen peroxide.

### Example 5. Analysis of the effect of PRDX6-KD (knockdown) hMSC on adhesion and spreading

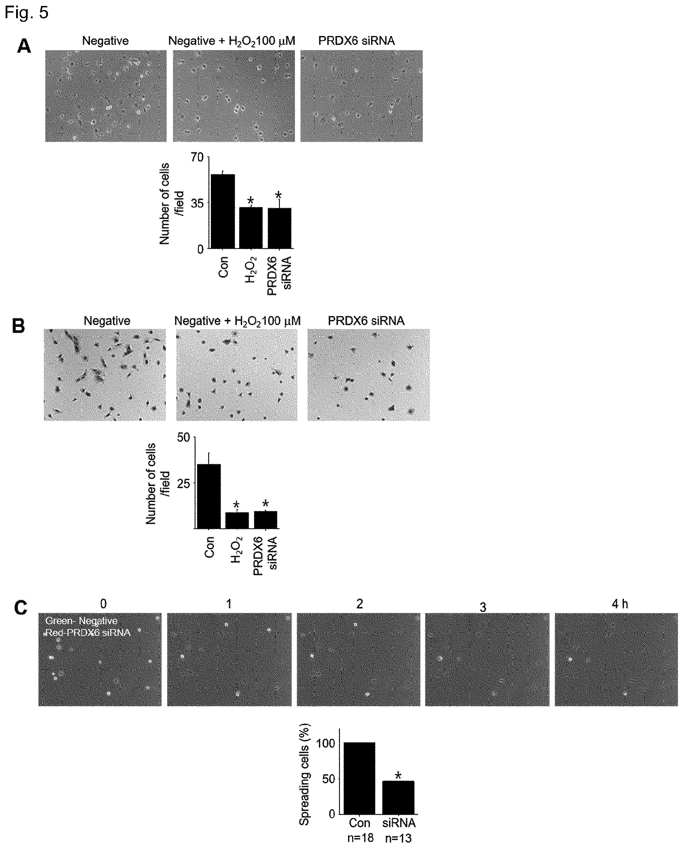

After treating siRNA to hMSC (2 × 10⁴ cells/well), it was added for 1 hour, and after washing with phosphate buffer saline (PBS), the cell number was analyzed through a microscope (A in FIG. 5). In addition, after treating siRNA to hMSC (2 × 10⁴ cells/well), it was added for 4 hours, and after washing with PBS, it was stained with crystal violet, and the number of cells was analyzed through a microscope (B in FIG. 5). The control group and PRDX6-KD hMSC were stained with CMFDA (green) and CMTMR (red), respectively. The hMSC (0.2 × 10⁵ cells/well) was added to a culture dish (Culture-Dish^{35mm}), and time-lapse imaging was filmed for 4 hours at 2-minute intervals. Snapshots of movies were taken for each time period to observe the adhesion and spreading of hMSC (C in FIG. 5).

Proliferation occurs by hMSC spreading after adhesion. In consideration of this characteristic, adhesion and spreading assays of hMSC were performed. After adding hMSC, cell adhesion was confirmed after 1 hour, and cell spreading was observed through crystal violet staining after 4 hours. As a result, it was confirmed that the hMSC in which PRDX6 was knocked down and the hMSC treated with hydrogen peroxide showed decreased cell adhesion (A in FIG. 5) and expansion (B in FIG. 5) compared to the control group. In addition, the control group hMSC and hMSC in which PRDX6 was knocked down were stained with CMFDA (green) and CMTPX (red) for 4 hours, respectively, and as a result, it was confirmed that the hMSC in which PRDX6 was knocked down showed decreased adhesion and spreading compared to the control group (C in FIG. 5). In conclusion, through this experiment, it was confirmed that PRDX6 affects spreading after hMSC adhesion.

### Example 6. Analysis of the effect of PRDX6-KD (knockdown) hMSC on motility and invasion

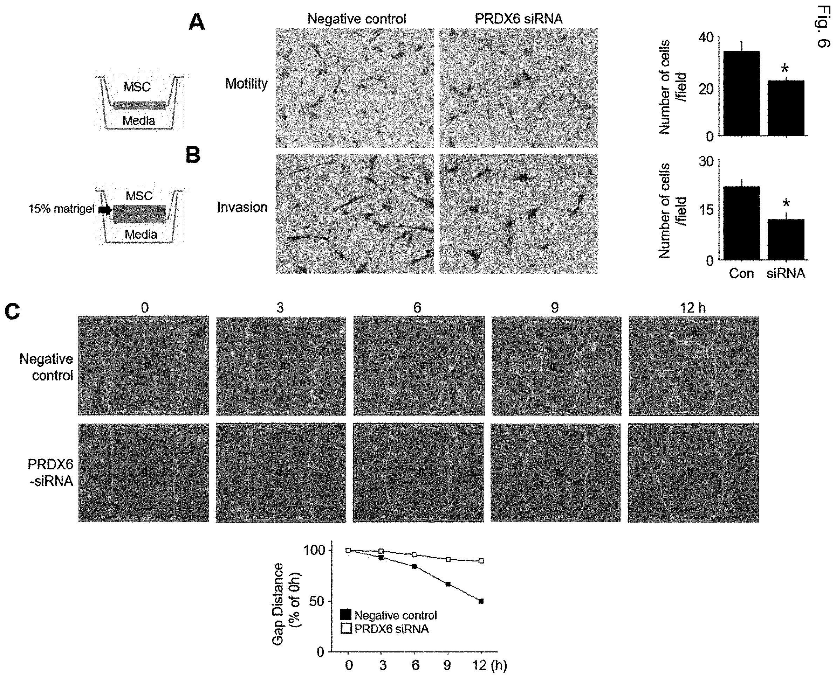

After treating siRNA to hMSC (1 × 10⁴ cells/well), it was added to a transwell plate for 24 hours, and crystal violet staining was performed to analyze motility (A in FIG. 6). In addition, after coating 15% Matrigel on a transwell plate for 24 hours, hMSC (1 × 10⁴ cells/well) was seeded for 24 hours, and crystal violet staining was performed to analyze invasion. (B in FIG. 6). Subsequently, hMSC (70 µL of 3 × 10⁵ cells/mL) was seeded using a Culture-insert m-Dish^{35mm} culture dish, and time-lapse imaging was filmed for 12 hours. Then, snapshots of movies were taken for each time period to measure the range of the box and confirm the movement of the hMSC (C in FIG. 6).

The hMSC was added to the upper well of the transwell for 24 hours, and a medium containing 10% FBS was added to the lower well. As a result of the experiment, it was confirmed that the hMSC in which PRDX6 was knocked down showed a decrease in cell motility compared to the control group (A in FIG. 6). In addition, in order to create an invasion environment, 15% Matrigel was coated in the upper well for 24 hours before the experiment, and then, the experiment was conducted. It was confirmed that the hMSC in which PRDX6 was knocked down showed a decrease in the invasion of cells compared to the control group (B in FIG. 6). As a result of confirming the movement of hMSC through imaging, it was confirmed that the hMSC in which RDX6 was knocked down showed a decrease in cell movement compared to the control group (C in FIG. 6).

### Example 7. Analysis of the effect of PRDX6-KD (knockdown) hMSC on migration

An experiment was conducted to determine if PRDX6 had an effect on the migration of hMSC. After siRNA was treated to hMSC (1 × 10⁴ cells/well), hMSC was added to the upper well of the transwell plate, and CXCL10 was added to the lower well. After 24 hours, crystal violet staining was performed for analysis.

Chemokine is a protein that regulates cell migration, and thus, a migration assay for CXCL10 matching CXCR3 expressed in hMSC was performed. After adding hMSC to the upper well of the transwell for 24 hours, recombinant CXCL10 was added to the lower well. As a result of the experiment, it was confirmed that the hMSC in which PRDX6 was knocked down showed a decrease in cell migration compared to the control group (FIG. 7).

### Example 8. Mechanism studies of PRDX6-KD (knockdown) hMSC

After siRNA was treated to hMSC, it was added for 48 hours, and the mRNA level and the protein level of hMSC were analyzed through RT-PCR and western blot, respectively. The mRNA levels of integrin and MMP of hMSC were analyzed via RT-PCR (A in FIG. 8), and the expression levels of integrin β3 and Rac1 of hMSC were analyzed through western blot (B and C in FIG. 8). In addition, the expression levels of ERK, p38, and JNK of hMSC were analyzed through western blot (D in FIG. 8).

Cell integrins are a group of cell membrane receptors known to play an important role in cell adhesion, migration, and proliferation. Integrin is a dimer composed of two units, α4β1 and α5β3, and when attached to the extracellular matrix, various types of biochemical signaling are induced. In addition, matrix metalloproteinase (MMP) of cells is a zinc-dependent neutral protease, which is known to play an important role in the metastasis of cancer by playing a role in the degradation of collagen, and an experiment was conducted to determine whether the motility of hMSC in which PRDX6 was knocked down was related to integrin and MMP.

As a result of RT-PCR (A in FIG. 8) and western blot (B in FIG. 8) analysis, it was confirmed that the hMSC in which PRDX6 was knocked down showed a decrease in integrin β3 of cells compared to the control group. However, there was no change in the expression level of MMP (A in FIG. 8). When attached to integrin, Rac1 protein is activated through stimulation to induce membrane protrusion toward the front of the cell to induce cell migration. As a result of the western blot analysis, it was confirmed that the hMSC in which PRDX6 was knocked down showed a decrease in Rac1 protein of cells compared to the control group (C in FIG. 8). In addition, integrin is also known to affect cell growth and proliferation in relation to the mitogen-activated protein kinase (MAPK) signaling system in the cytoplasm. As a result of the western blot analysis, it was confirmed that the phosphorylation of ERK in the MAPK of cells was reduced in the hMSC in which PRDX6 was knocked down, compared to the control group (D in FIG. 8). In conclusion, through this experiment, it can be seen that PRDX6 regulates integrin β3 of hMSC, and thereby affects Rac1-ERK signaling, and thus, it is involved in cell motility and also cell proliferation.

### Example 9. Analysis of the effect of PRDX6-KD (knockdown) hMSC on immunosuppressive capacity

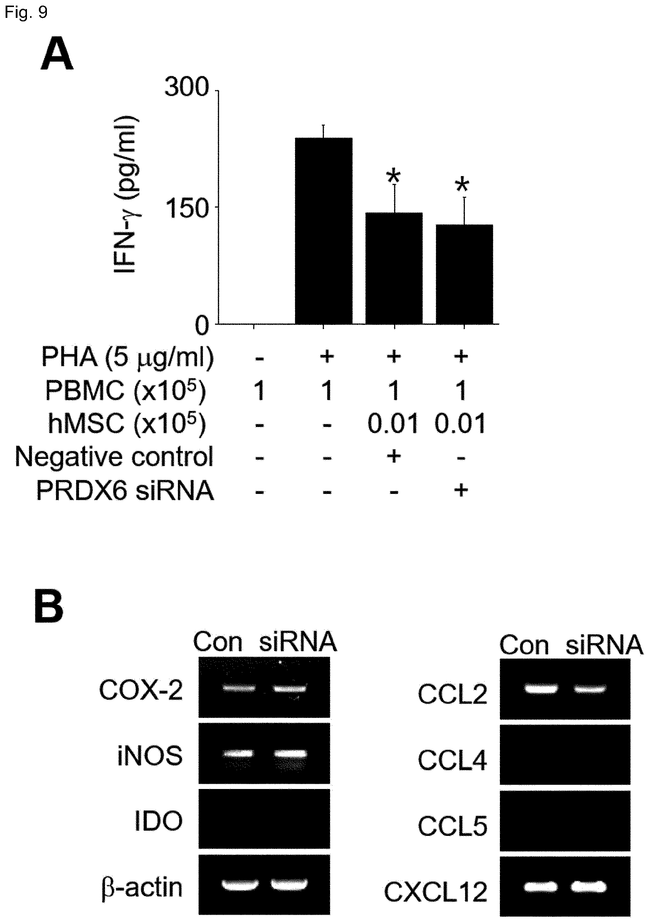

The hMSC is known to inhibit the function of T cells, and an experiment was conducted to determine if PRDX6 also affects the immunosuppressive capacity of hMSC. After siRNA was treated to hMSC, the hMSC and peripheral blood mononuclear cells (PBMC) were co-cultured with phytohemagglutinin (PHA, 5 ug /mL) for 72 hours, and then, the protein level of IFN-γ in the supernatant was analyzed by ELISA (A in FIG. 9). In addition, the total RNA was isolated using TRIzol for hMSC, and then, gene expression was confirmed through RT-PCR (B in FIG. 9).

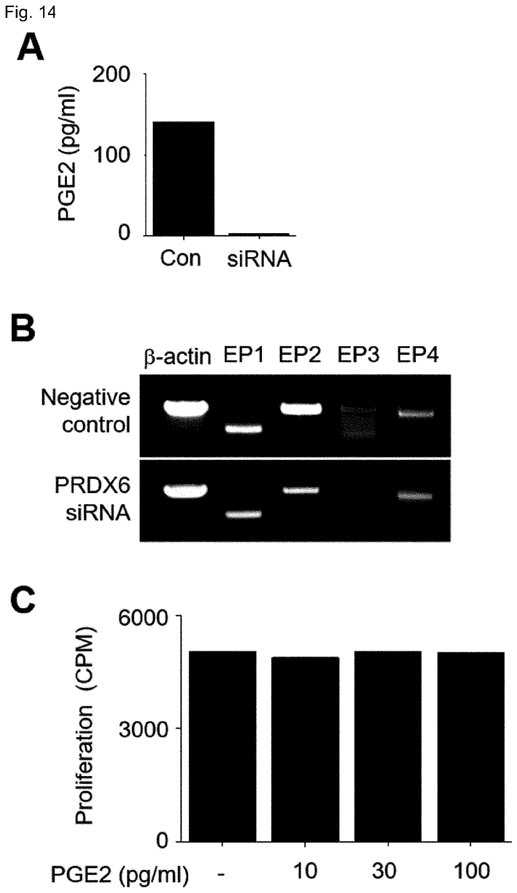

As a result of the experiment, it was confirmed that the hMSC in which PRDX6 was knocked down inhibited IFN-λ secretion of T cells as in the control group hMSC (A in FIG. 9). In addition, as a result of confirming the expression levels of COX-2, NO, IDO, which are soluble factors involved in immunomodulation in hMSC, and chemokine proteins CCL2, CCL4, CCL5, and CXCL12, it was confirmed that there was no difference between the two cell groups (B in FIG. 14). That is, through this experiment, it was confirmed that PRDX6 is not involved in the immunosuppressive function of hMSC.

### Example 10. Analysis of characteristics of PRDX6-transgenic mMSC

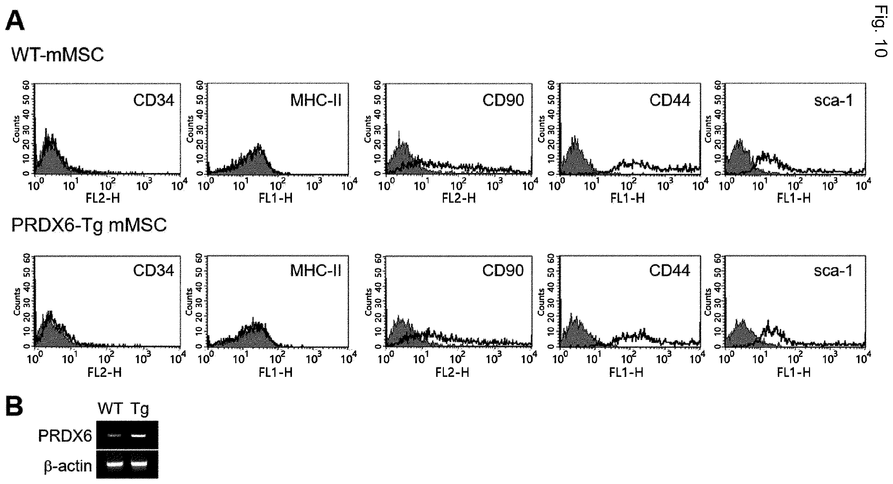

The function of PRDX6 was verified using mouse MSC (mMSC) overexpressing PRDX6. After bone marrow was separated from PRDX6 overexpressing transgenic (Tg) mice and cultured for 16 days, the cell phenotype was analyzed through FACS (A in FIG. 10). After the total RNA was isolated using TRIzol in mMSC, gene expression was confirmed through RT-PCR (B in FIG. 9). As a result of analysis, there was no difference in the phenotype of wild-type (WT)-mMSC and PRDX6-Tg mMSC (A in FIG. 10). In addition, by RT-PCR analysis, it was confirmed that PRDX6-Tg mMSC overexpresses PRDX6 compared to mMSC of the control group (B in FIG. 10).

### Example 11. Analysis of the effect of PRDX6-transgenic mMSC on cell proliferation

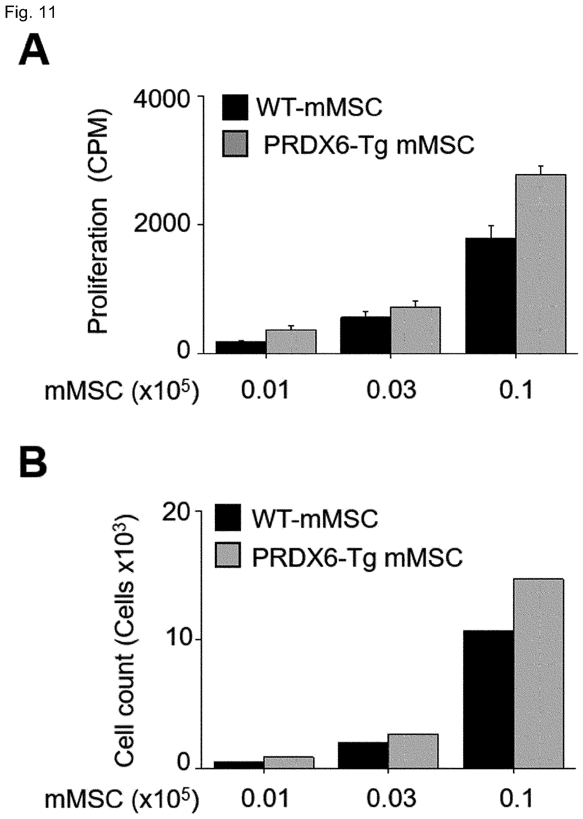

After adding mMSC to a 96-well plate, the cells were cultured for 72 hours, and cell proliferation was measured through a thymidine uptake (A in FIG. 11) and cell count (B in FIG. 11). As a result of the measurement, it was confirmed that PRDX6-Tg mMSC showed an increase in cell proliferation compared to the control group (A and B in FIG. 11). That is, it was confirmed through the experiment that PRDX6 of mMSC has an effect on cell proliferation.

### Example 12. Analysis of the effect of PRDX6-transgenic mMSC on motility and invasion

An experiment was conducted to determine if PRDX6 also affected motility, invasion, and migration of mMSC.

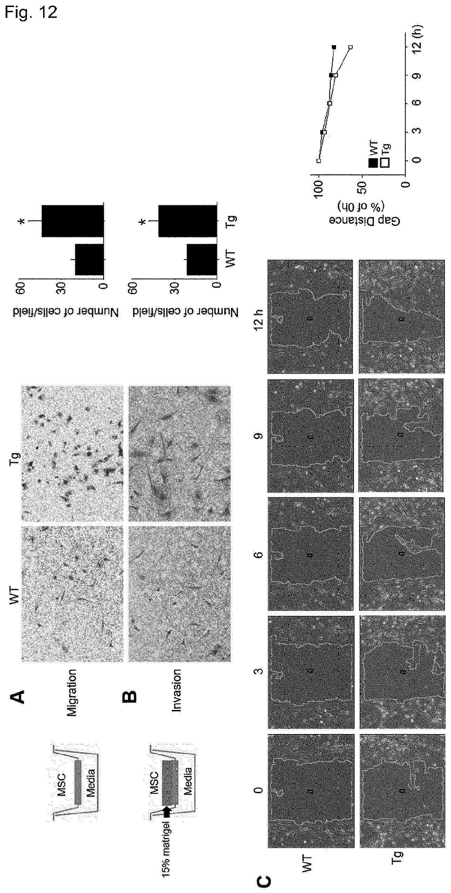

After adding mMSC (5 × 10⁴ cells/well) to a transwell plate for 24 hours, crystal violet staining was performed for analysis (A in FIG. 12). After coating 15% Matrigel on a transwell plate for 24 hours, mMSC (5 × 10⁴ cells/well) was added for 24 hours, and crystal violet staining was performed for analysis. (B in FIG. 12). Using a Culture-insert m-Dish^{35mm} culture dish, mMSC (70 µL of 3 × 10⁵ cells/mL) was seeded and time-lapse imaging was filmed for 12 hours. Then, snapshots of movies were taken for each time period to measure the range of the box and confirm the movement of the hMSC (C in FIG. 12).

In order to measure motility, mMSC was added to the upper well of the transwell for 24 hours, and a medium containing 10% FBS was added to the lower well. As a result of the measurement, it was confirmed that PRDX6-Tg mMSC showed an increase in the motility of cells compared to the control group (A in FIG. 12). In addition, after 15% Matrigel was coated on the upper well for 24 hours, invasion assay was performed. It was confirmed that PRDX6-Tg mMSC showed an increase in the invasion compared to the control group (B in FIG. 12). Afterwards, as a result of confirming the movement of mMSC through imaging, it was confirmed that PRDX6-Tg mMSC showed an increase in the movement of cells compared to the control group (C in FIG. 12). That is, through this experiment, it was confirmed that PRDX6 overexpression increases the movement of mMSC.

### Example 13. Analysis of the effect of PRDX6-transgenic mMSC on immunosuppressive capacity

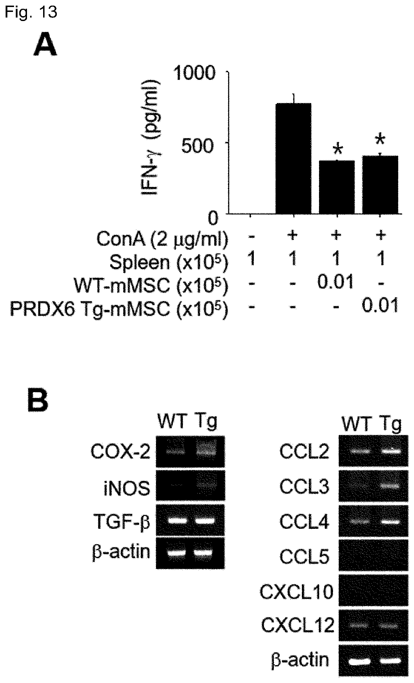

An experiment was conducted to determine if PRDX6 affects the immunosuppressive capacity of mMSC. After 72 hours of co-culturing mouse spleen cells with mMSC with concanavalin A (ConA), the protein level of IFN-γ in the supernatant was analyzed by ELISA (A in FIG. 13). After the total RNA was isolated using TRIzol in mMSC, gene expression was confirmed through RT-PCR (B in FIG. 13). As a result of the experiment, it was confirmed that PRDX6-Tg mMSC inhibits the secretion of IFN-λ of T cells, similar to the control group hMSC (A in FIG. 13). In addition, as a result of confirming the expression levels of COX-2, NO, IDO, which are soluble factors involved in immunomodulation in mMSC, and chemokine proteins CCL2, CCL4, CCL5, CXCL10, and CXCL12, it was confirmed that there was no difference between the two cell groups (B in FIG. 13). That is, it was confirmed that PRDX6 does not affect the immunosuppressive capacity of mMSC.

### Example 14. Analysis of association of PRDX6-knockdown hMSC and PGE2

PRDX6 is known as a bifunctional protein. PRDX6 has a function of removing hydrogen peroxide and a function of activating calcium-independent phospholipase A2 (iPLA2), which produces prostaglandin E2 (PGE2) from arachidonate. In order to confirm the association between PRDX6-knockdown hMSC and PGE2, after treating siRNA to hMSC, the protein level of PGE2 in the supernatant was analyzed by ELISA (A in FIG. 14). After the total RNA was isolated from hMSC using TRIzol, gene expression was confirmed through RT-PCR (B in FIG. 14). Then, after hMSC was added, PGE2 was treated, and cell proliferation was analyzed through a thymidine uptake after 72 hours (C in FIG. 14).

As a result of the analysis using ELISA, it was confirmed that the hMSC in which PRDX6 was knocked down showed a decrease in PGE2 of cells compared to the control group (A in FIG. 14). In addition, as a result of RT-PCR analysis, it was confirmed that hMSC expressed EP1, EP2, and EP4 among prostaglandin E2 receptors (EPs), which are PGE2 receptors (B in FIG. 14). In addition, after directly treating PGE2 to hMSC, cell proliferation was measured, but it was confirmed that PGE2 does not affect the proliferation of hMSC (C in FIG. 14). In conclusion, it was confirmed that while PRDX6 produces PGE2 through iPLA2 activity, it does not have a direct association with cell proliferation.

The above description of the present invention is for illustration only, and those skilled in the art to which the present invention pertains can understand that the present invention can be easily modified into other specific forms without changing the technical spirit or essential features of the present invention. Therefore, it should be understood that the exemplary embodiments described above are illustrative in all respects and not restrictive.

## Claims

1. A biomarker for prediction of proliferation and migration capacity of mesenchymal stem cells, comprising peroxiredoxin 6.

2. The biomarker of claim 1, wherein the peroxiredoxin 6 regulates integrin β3 of mesenchymal stem cells.

3. The biomarker of claim 1, wherein cell proliferation and migration capacity are increased when the peroxiredoxin 6 is overexpressed.

4. A composition for prediction of proliferation and migration capacity of mesenchymal stem cells, comprising a agent for measuring the expression level of the biomarker of claim 1.

5. The composition of claim 4, wherein the agent for measuring the expression level of the biomarker comprises a primer pair, a probe or an antisense nucleotide that specifically binds to the biomarker.

6. A kit for prediction of proliferation and migration capacity of mesenchymal stem cells, comprising the composition of claim 4.

7. The kit of claim 6, wherein the kit is an RT-PCR kit, a competitive RT-PCR kit, a real-time RT-PCR kit, a quantitative RT-PCR kit, or a DNA chip kit.

8. A method for providing information for prediction of proliferation and migration capacity of mesenchymal stem cells, comprising measuring the expression level of the biomarker of claim 1 from a biological sample of a subject to compare with the expression level of the corresponding biomarker of a comparative control group sample.

9. The method of claim 8, wherein the method for measuring the expression level of the biomarker is reverse transcriptase polymerase chain reaction (RT-PCR), competitive reverse transcriptase polymerase chain reaction (competitive RT-PCR), real-time quantitative reverse transcriptase polymerase chain reaction (real-time quantitative RT-PCR), quantitative RT-PCR, an RNase protection method, Northern blotting or DNA chip technology, Northern blotting, immunohistochemical staining, immunoprecipitation assay, complement fixation assay, or immunofluorescence.

10. A method for screening mesenchymal stem cells with excellent cell proliferation and migration capacity, by measuring the expression level of peroxiredoxin 6.
